# EUROPEAN PATENT APPLICATION

(11) **EP 4 692 309 A1**
(43) Date of publication of application: **11.02.2026**
(21) Application number: 24780777.9
(22) Date of filing: 29.03.2024
(51) Int. Cl.: C12N 1/20, A23L 33/135, C12N 15/31, C12N 15/56

(54) **NOVEL BACTERIUM BELONGING TO GENUS BIFIDOBACTERIUM**

(30) Priority: 31.03.2023 JP 2023058435
(71) Applicant: Kabushiki Kaisha Yakult Honsha, Tokyo, 105-8660 (JP)
(72) Inventor: WATANABE, Yohei, Tokyo 105-8660 (JP); SHIBATA, Junko, Tokyo 105-8660 (JP); KASAHARA, Kohei, Tokyo 105-8660 (JP); MORITA, Yuki, Tokyo 105-8660 (JP); GOMI, Atsushi, Tokyo 105-8660 (JP)
(74) Representative: Wächtershäuser & Hartz Patentanwaltspartnerschaft mbB
(86) International application number: PCT/JP2024/012978
(87) International publication number: WO 2024/204682

(57) **Abstract**

Provided is a bacterium belonging to *Bifidobacterium pseudocatenulatum* which is capable of utilizing xylans and is excellent in viability in a milk component-containing medium. The present invention provides a bacterium belonging to *Bifidobacterium pseudocatenulatum,* the bacterium having xylanase gene on the genome and having higher viability in a milk component-containing medium than *Bifidobacterium pseudocatenulatum* YIT 11057 (NITE BP-02930).

## Description

### Technical Field

The present invention relates to a bacterium belonging to the genus *Bifidobacterium* which is classified into *Bifidobacterium pseudocatenulatum.*

### Background Art

One of factors of probiotics influencing physiological functions of hosts is presumed to be that probiotics metabolize carbohydrate sources remaining in the digestive tract and produce short-chain fatty acids such as lactic acid and acetic acid. Previous studies have revealed that the genome of a bifidobacterial species dominant in the intestinal tracts of adults is rich in genes involved in the metabolism of indigestible polysaccharides derived from plants, particularly, xylans abundantly contained in diets for adults, as compared with the genomes of bifidobacterial species derived from infants.

The present applicant previously found a bacterium belonging to the genus *Bifidobacterium* which has xylanase gene on the genome and can efficiently utilize xylans (Patent Literature 1). This bacterium belonging to the genus *Bifidobacterium* is expected to proliferate in the intestines of adults and to have a high ability to produce organic acids and is therefore a promising candidate for probiotics intended for adults.

### Citation List

### Patent Literature

Patent Literature 1: WO 2020/203782

### Summary of Invention

### Technical Problem

*Bifidobacterium pseudocatenulatum* YIT 11057 described in Patent Literature 1 has xylanase gene and is expected to actively metabolize carbohydrates in the intestines and produce organic acids. However, in consideration of use in fermented milk production, YIT 11057 has been found to not always have sufficient viability in a milk component-containing medium. Accordingly, the present invention relates to provision of a novel bacterium belonging to *Bifidobacterium pseudocatenulatum* which is capable of utilizing xylans and exhibits excellent viability in a milk component-containing medium.

### Solution to Problem

The present inventors conducted diligent studies and consequently obtained a novel bacterium belonging to *Bifidobacterium pseudocatenulatum* which can efficiently utilize xylans and exhibits excellent viability in a milk component-containing medium.

Specifically, the present invention provides the following [1] to [6].
[1] A bacterium belonging to *Bifidobacterium pseudocatenulatum,* the bacterium having xylanase gene on a genome and having higher viability in a milk component-containing medium than *Bifidobacterium pseudocatenulatum* YIT 11057 (NITE BP-02930).
[2] The bacterium according to [1], wherein the xylanase gene is a polynucleotide consisting of the nucleotide sequence represented by SEQ ID NO: 1 or a polynucleotide having 70% or more identity to the nucleotide sequence and encoding a protein having xylanase activity.
[3] The bacterium according to [1], wherein a survival rate is 20% or more when the bacterium is cultured in the milk component-containing medium for 24 hours and then preserved at a low temperature for 2 weeks.
[4] *Bifidobacterium pseudocatenulatum* Y 51493 (NITE ABP-03852), *Bifidobacterium pseudocatenulatum* YIT 13179 (NITE ABP-03853), or a bacterial strain closely related thereto.
[5] A food or drink product comprising the bacterium according to any one of [1] to [4].
[6] The food or drink product according to [5] which is a fermented milk food or drink product.

### Advantageous Effects of Invention

The novel bacterium belonging to *Bifidobacterium pseudocatenulatum* according to the present invention has an ability to degrade and utilize xylans, particularly, arabinoxylan, abundantly contained in diets for adults, and exhibits excellent viability in a milk component-containing medium. Thus, the bacterium of the present invention is expected to proliferate in the intestines of adults and to have a high ability to produce organic acids and as such, can be used as probiotics for adults in medicaments, food or drink products, and the like.

### Brief Description of Drawings

Figure 1 illustrates change in the number of viable bacteria at the time of preservation of Y 51493 and YIT 11057 cultured in a milk component-containing medium.
Figure 2 illustrates change in survival rate at the time of preservation of Y 51493 and YIT 11057 cultured in a milk component-containing medium.
Figure 3 illustrates one example of the genomic structure of a strain that acquired xylanase gene (transformant) of *Bifidobacterium pseudocatenulatum.* Corresponding single-nucleotide polymorphisms (SNPs) of each strain are indicated by gray line.
Figure 4 illustrates the proliferation curves of a donor (*Bifidobacterium pseudocatenulatum* YIT 11027), a recipient (*Bifidobacterium pseudocatenulatum* YIT 11956), and the strain that acquired the gene (transformant) in mPY-AX medium.
Figure 5 illustrates the proliferation curves of a donor (*Bifidobacterium pseudocatenulatum* YIT 11027), a recipient (*Bifidobacterium pseudocatenulatum* YIT 11956), and the strain that acquired the gene (transformant) in mPY-Starch medium.
Figure 6 illustrates the colony formation of a donor (*Bifidobacterium pseudocatenulatum* YIT 4072^{T}), a recipient (*Bifidobacterium pseudocatenulatum* YIT 12824), and the strain that acquired the gene (transformant) in Tc-mGAML medium.
Figure 7 illustrates transformation frequencies when (A) viable bacterial cells or dead bacterial cells, (B) viable bacterial cells, genomic DNA, or PCR products, and (C) viable bacterial cells treated with DNase were used as donors.
Figure 8 illustrates change in the number of viable bacteria at the time of preservation of YIT 13179 and Y 51493 cultured in a milk component-containing medium, and their survival rates on preservation day 28.

### Description of Embodiments

The bacterium of the present invention is a bacterium belonging to *Bifidobacterium pseudocatenulatum* which has xylanase gene on the genome and exhibits excellent viability in a milk component-containing medium. In one aspect, the bacterium of the present invention is a bacterium belonging to *Bifidobacterium pseudocatenulatum,* the bacterium having xylanase gene on the genome and having higher viability in a milk component-containing medium than *Bifidobacterium pseudocatenulatum* YIT 11057 (NITE BP-02930). YIT 11057 has the xylanase gene on the genome. YIT 11057 was deposited on March 25, 2019 at the National Institute of Technology and Evaluation, Patent Microorganisms Depositary (#122, 2-5-8 Kazusakamatari, Kisarazu-shi, Chiba, Japan).

"Xylanase gene" refers to a gene encoding xylanase classified into GH10 (endo-1,4-beta-xylanase A; xynA). "Xylanase gene" possessed on the genome by the bacterium belonging to *Bifidobacterium pseudocatenulatum* according to the present invention specifically encompasses a polynucleotide consisting of the nucleotide sequence represented by SEQ ID NO: 1 or a polynucleotide having 70% or more, preferably 90% or more, more preferably 95% or more, more preferably 98% or more, more preferably 99% or more identity to the nucleotide sequence and encoding a protein having xylanase activity.

In this context, the polynucleotide consisting of the nucleotide sequence represented by SEQ ID NO: 1 refers to xylanase gene possessed by YIT 11057.

The identity of nucleotide sequences is a value which is obtained by aligning both the nucleotide sequences such that the two nucleic acid sequences to be compared are in match with each other as much as possible, dividing the number of matched bases by the total number of bases, and expressing the quotient in percentage. Those skilled in the art can appropriately set parameters of software such as BLAST, ClustalX, or Genetyx to determine the identity of the nucleotide sequences.

The xylanase activity means activity of hydrolyzing the xylose backbone of xylans with xylans as a substrate to produce xylooligosaccharide and xylose (xylan hydrolytic activity).

In this context, examples of the xylans include xylan, arabinoxylan, glucuronoxylan, glucuronoarabinoxylan, and acetylxylan. Xylan and arabinoxylan are preferred.

Thus, the bacterium belonging to *Bifidobacterium pseudocatenulatum* having the xylanase gene on the genome is, in other words, a bacterium belonging to *Bifidobacterium pseudocatenulatum* having an ability to degrade the xylose backbone of xylans.

As used herein, "having the ability to utilize xylans" refers to having an ability to proliferate with xylans as a carbon source. Specifically, this phrase refers to, for example, being able to proliferate in a medium containing xylans as only one carbon source. For example, this phrase means that turbidity, for example, in terms of increase in OD₆₀₀, is 0.1 or more when culture is performed in a medium containing only xylans as a carbohydrate source for 72 hours. The turbidity is preferably 0.3 or more, more preferably 0.4 or more, in terms of increase in OD₆₀₀. If change in turbidity cannot be measured, for example, due to a medium supplemented with xylans rich in insoluble fractions, the phrase means that the amount of short-chain fatty acids produced, for example, the total amount of lactic acid, acetic acid, and formic acid produced, is 10 mM or more in a culture supernatant obtained by culture for 72 hours. The amount of short-chain fatty acids produced is preferably 20 mM or more, more preferably 40 mM or more in total.

A carbohydrate source-free medium supplemented with xylans can be used as a culture medium for use in a test of the ability to utilize xylans. The composition of the carbohydrate source-free medium can be the composition of M-ILS medium, Peptone-Yeast (PY) medium, or the like.

The amount of the xylans added to the medium is preferably from 0.01 to 10% by mass, more preferably from 0.05 to 5% by mass, further more preferably from 0.1 to 1% by mass, in the medium. For a control, it is desirable to culture the target bacterium belonging to *Bifidobacterium pseudocatenulatum* in the carbohydrate source-free medium and confirm the OD value.

"Milk component" means a material containing a component derived from milk including raw milk of animal milk such as cow milk, goat milk, or sheep milk, heated milk, skim milk powder, whole milk powder, fresh cream, or whey. Among them, skim milk powder is preferred. "Whey" is obtained by removing fat and casein from milk.

"Milk component-containing medium" may not be limited as long as the medium contains a milk component. The milk component-containing medium may further contain an additional component such as carbohydrates, vitamins, protein hydrolysates, amino acids, minerals, salts, surfactants, fatty acids, or metals. The additional component is not particularly limited and is preferably carbohydrates such as glucose, galactose, lactose, or fructose.

The content of the milk component in the medium is not particularly limited and is preferably from 1 to 50% by mass (hereinafter, the simple term "%" means % by mass), more preferably from 2 to 10%, based on a solid content.

"Viability in milk component-containing medium" indicates to how many viable bacteria exist when the target bacterium belonging to *Bifidobacterium pseudocatenulatum* is cultured in the milk component-containing medium for 24 hours and then preserved at a low temperature for 2 weeks. The number of viable bacteria can be determined in accordance with a routine method. The number of viable bacteria in the milk component-containing medium can be determined by, for example, a colony count method. Also, a survival rate can be represented by the ratio of the number of viable bacteria after preservation at a low temperature for 2 weeks to the number of viable bacteria immediately after culture for 24 hours. In this context, the low temperature refers to a temperature zone usually used in preserving microbes, and is preferably 10°C or lower.

"Higher viability in milk component-containing medium than *Bifidobacterium pseudocatenulatum* YIT 11057" more specifically means that a survival rate is preferably 20% or more, more preferably 40% or more, further more preferably 50% or more, when bacterium belonging to *Bifidobacterium pseudocatenulatum* is cultured in the milk component-containing medium for 24 hours and then preserved at a low temperature for 2 weeks.

The bacterium belonging to *Bifidobacterium pseudocatenulatum* according to the present invention has the xylanase gene on the genome and has the ability to degrade and utilize xylans and can therefore contribute to the supply of short-chain fatty acids such as acetic acid, lactic acid, and formic acid produced by the degradation of xylans. The bacterium belonging to *Bifidobacterium pseudocatenulatum* according to the present invention has high viability in a milk component-containing medium and can therefore effectively exert physiological effects of the bacterium and is useful in the production of medicaments, food or drink products, and the like.

The bacterium belonging to *Bifidobacterium pseudocatenulatum* according to the present invention can be obtained, for example, by screening bacteria belonging to *Bifidobacterium pseudocatenulatum* present in the intestines of human (e.g., adults or infants), with the presence of the xylanase gene or the ability to utilize xylans and the viability in a milk component-containing medium mentioned above as an index. Alternatively, the bacterium belonging to *Bifidobacterium pseudocatenulatum* according to the present invention can be obtained, for example, by screening bacteria prepared by a known breeding method using, as a parent strain, an arbitrary bacterium belonging to *Bifidobacterium pseudocatenulatum* such as a bacterium belonging to *Bifidobacterium pseudocatenulatum* present in the intestines of human (e.g., adults or infants); screening bacteria prepared by subjecting the parent strain with the treatment with ultraviolet irradiation or a mutagenesis inducer such as nitrosoguanidine (NTG) or ethyl methanesulfonate (EMS); or screening bacteria prepared by altering the genome of the parent strain by a known site-directed mutagenesis or the lie, with the presence of the xylanase gene or the ability to utilize xylans and the viability in a milk component-containing medium mentioned above as an index. Alternatively, the bacterium belonging to *Bifidobacterium pseudocatenulatum* according to the present invention can be obtained, for example, by screening bacteria prepared by a method for transforming a bacterium belonging to the genus *Bifidobacterium* mentioned in detail in Reference Example 1 or 2 described later using, as a recipient (parent strain), an arbitrary bacterium belonging to *Bifidobacterium pseudocatenulatum* such as a bacterium belonging to *Bifidobacterium pseudocatenulatum* present in the intestines of human (e.g., adults or infants), with the presence of the xylanase gene or the ability to utilize xylans and the viability in a milk component-containing medium mentioned above as an index. In this context, the parent strain is preferably a bacterium belonging to *Bifidobacterium pseudocatenulatum* having the xylanase gene on the genome, more preferably YIT 11057.

As shown in Example 1 described later, the present inventors bred YIT 11057 as a parent strain by UV irradiation and long-term subculture to obtain Y 51493 having higher viability in a milk component-containing medium than YIT 11057. As shown in Example 2 described later, Y 51493 was transformed to obtain YIT 13179 having equivalent viability in milk component-containing medium to Y 51493. The bacterium belonging to *Bifidobacterium pseudocatenulatum* according to the present invention is not limited thereto as long as the bacterium has the xylanase gene on the genome and has higher viability in a milk component-containing medium than YIT 11057. The bacterium belonging to *Bifidobacterium pseudocatenulatum* encompasses bacterial strains closely related to these strains biologically and genetically. The bacterium belonging to *Bifidobacterium pseudocatenulatum* according to the present invention may be a natural strain present in nature or may be a mutant or a genetically modified strain of the natural strain as long as the bacterium has the xylanase gene on the genome and has higher viability in a milk component-containing medium than YIT 11057.

Y 51493 and YIT 13179 were deposited on March 13, 2023 at the National Institute of Technology and Evaluation, Patent Microorganisms Depositary (#122, 2-5-8 Kazusakamatari, Kisarazu-shi, Chiba, Japan), as shown below.
*Bifidobacterium pseudocatenulatum* Y 51493 (NITE ABP-03852), and
*Bifidobacterium pseudocatenulatum* YIT 13179 (NITE ABP-03853).

Among them, YIT 13179 or its closely related bacterial strain is preferred from the viewpoint of the ability to utilize xylans, from the viewpoint of viability in a milk component-containing medium, and from the viewpoint of further having the ability to utilize glucose. The strain having the ability to utilize glucose is expected to produce organic acids from polysaccharides or oligosaccharides having glucose as a constituent saccharide.

In this context, the ability to utilize glucose refers to having the ability to proliferate with glucose as a carbon source.

The closely related bacterial strain refers to a bacterial strain having matched sequences in the multilocus sequence analysis (MLSA) method for evaluating a plurality of housekeeping gene sequences.

Specifically, the full-length sequences of DNA gyrase subunit B (gyrB), 50S ribosomal protein L2 (rplB), amidophosphoribosyltransferase (purF), DNA-directed RNA polymerase subunit beta (rpoB), ATP-dependent Clp protease ATP-binding subunit ClpC1 (clpC), elongation factor G (fusA), and isoleucine-tRNA ligase (ileS) reported as genes suitable for MLSA analysis on bifidobacteria (International Journal of Systematic and Evolutionary Microbiology (2006), 56, 2783-2792) are obtained, and all the genes are linked for each bacterial strain. Then, the sequence match between the strains to be compared is tested using a program such as VSEARCH or BLAST. When the sequence identity is 90% or more, preferably 95% or more, more preferably 98% or more, more preferably 99% or more, it can be determined that the bacterial strains are closely related species.

The nucleotide sequences of gyrB, rplB, purF, rpoB, clpC, fusA, and ileS in YIT 11057 are shown in the Sequence Listing (gyrB: SEQ ID NO: 2, rplB: SEQ ID NO: 3, purF: SEQ ID NO: 4, rpoB: SEQ ID NO: 5, clpC: SEQ ID NO: 6, fusA: SEQ ID NO: 7, ileS: SEQ ID NO: 8).

The application form of the bacterium belonging to *Bifidobacterium pseudocatenulatum* according to the present invention is not particularly limited, and may be freeze-dried or cultures containing the bacterium can be used. In any of the forms, the bacterium is preferably in the state of a viable bacterium.

The bacterium belonging to *Bifidobacterium pseudocatenulatum* according to the present invention may be mixed with a solid or liquid nontoxic carrier for a medicament and used in the form of a common pharmaceutical preparation. Examples of such a preparation include solid formulations such as tablets, granules, powders, and capsules, liquid formulations such as solutions, suspension, and emulsions, and freeze-dried preparations. These preparations can be prepared by usual pharmaceutical approaches. Examples of the nontoxic carrier for a medicament include glucose, lactose, sucrose, starch, mannitol, dextrin, fatty acid glyceride, polyethylene glycol, hydroxyethyl starch, ethylene glycol, polyoxyethylene sorbitan fatty acid ester, amino acids, gelatin, albumin, water, and saline. If necessary, a common additive such as a stabilizer, a wetting agent, an emulsifier, a binder, a tonicity agent, or an excipient may be appropriately added thereto.

The bacterium belonging to *Bifidobacterium pseudocatenulatum* according to the present invention may be not only prepared into the preparation as described above but blended into a food or drink product and used. The bacterium, when blended into a food or drink product, can be contained as it is or together with various nutrients. Specifically, in the case of blending the bacterium belonging to *Bifidobacterium pseudocatenulatum* according to the present invention into a food or drink product, the bacterium may be made into a form suitable for eating, i.e., granules, grains, tablets, capsules, a paste, or the like, by use of a common approach appropriately using an additive that may be used for food or drink products, or may be added for use to various food products, for example, meat processed food products such as ham and sausage, seafood processed food products such as Kamaboko (fish paste) and Chikuwa (fish sausage), bread, confectionery, butter, or powdered milk, or may be added for use to beverages such as water, fruit juice, milk, soft drinks, or tea beverages. The food or drink product also includes animal feed.

A fermented milk food or drink product or a fermented food or drink product such as fermented soymilk, fermented fruit juice, or fermented vegetable juice, containing the bacterium belonging to *Bifidobacterium pseudocatenulatum* according to the present invention in the state of a viable bacterium, is suitably used as the food or drink product. Particularly, a fermented milk food or drink product is preferably used. The fermented milk food or drink product can be produced in accordance with a routine method. For example, in the case of producing fermented milk, the bacterium belonging to *Bifidobacterium pseudocatenulatum* according to the present invention is inoculated to and cultured in a sterilized milk medium, either alone or together with another microbe, and the resultant is homogenized to obtain a fermented milk base. Next, a separately prepared syrup solution is added to and mixed with the base, and the mixture is homogenized with a homogenizer or the like. A flavor can be further added thereto to prepare a final product. The fermented milk food or drink product thus obtained may be prepared as a product in any form such as syrup (sweetener)-free plain type, soft type, fruit flavor type, solid state, or liquid state.

An arbitrary component such as a sweetener (e.g., a syrup), an emulsifier, a thickener (or a stabilizer), or various vitamins can be blended into such a fermented milk food or drink product. The following may be blended thereinto: as the syrup, carbohydrates such as glucose, sucrose, fructose, high-fructose corn syrup, glucose syrup, palatinose, trehalose, lactose, xylose, galactooligosaccharide (GOS), xylooligosaccharide (XOS), arabinoxylooligosaccharide (AXOS), xylan, arabinoxylan, arabinooligosaccharide (AOS), arabinan, maltose, honey, and molasses, sugar alcohols such as sorbitol, xylitol, erythritol, lactitol, palatinit, reduced sugar syrup, and reduced maltose syrup, high-intensity sweeteners such as aspartame, thaumatin, sucralose, acesulfame K, and stevia, emulsifiers such as glycerin fatty acid ester, polyglycerin fatty acid ester, sorbitan fatty acid ester, and lecithin, and thickeners (or stabilizers) such as agar, gelatin, carrageenan, guar gum, xanthan gum, pectin, locust bean gum, gellan gum, carboxymethylcellulose, soybean polysaccharides, and propylene glycol alginate. In addition, the following may be blended thereinto: vitamins such as vitamin A, vitamin B, vitamin C, and vitamin E, minerals such as calcium, magnesium, zinc, iron, and manganese, acidulants such as citric acid, lactic acid, acetic acid, malic acid, tartaric acid, and gluconic acid, milk fats such as cream, butter, and sour cream, flavors such as yogurt, berry, orange, quince, perilla, citrus, apple, mint, grape, apricot, pear, custard cream, peach, melon, banana, tropical, herb, black tea, and coffee, herb extracts, brown sugar extracts, and the like.

In the production of the fermented milk food or drink product, a microbe other than the bacterium belonging to *Bifidobacterium pseudocatenulatum* according to the present invention may be used in combination therewith. Examples of such a microbe include bacteria belonging to the genus *Lacticaseibacillus* such as *Lacticaseibacillus paracasei,* bacteria belonging to the genus *Lactobacillus* such as *Lactobacillus casei, L. acidophilus, L. plantarum, L. buchneri, L. gallinarum, L. amylovorus, L. brevis, L. rhamnosus, L. kefir, L. paracasei, L. crispatus, L. zeae, L. helveticus, L. salivalius, L. gasseri, L. fermentum, L. reuteri, L. delbrueckii* subsp. *bulgaricus, L. delbrueckii* subsp. *delbrueckii,* and *L. johnsonii,* bacteria belonging to the genus *Streptococcus* such as *Streptococcus thermophilus,* bacteria belonging to the genus *Lactococcus* such as *Lactococcus lactis* subsp. *lactis* and *Lactococcus lactis* subsp. *cremoris,* bacteria belonging to the genus *Enterococcus such as Enterococcus faecalis and E. faecium,* bacteria belonging to the genus *Bacillus* such as *Bacillus subtilis,* and yeasts belonging to the genera *Saccharomyses, Torulaspora,* and *Candida* such as *Saccharomyses cerevisiae, Torulaspora delbrueckii,* and *Candida kefyr.* The bacterium belonging to *Bifidobacterium pseudocatenulatum* according to the present invention is preferably used in combination with one or more members selected from the group consisting of a bacterium belonging to the genus *Lactobacillus,* a bacterium belonging to the genus *Streptococcus,* and a bacterium belonging to the genus *Lactococcus* to produce a fermented milk food or drink product because high palatability is obtained and facilitates drinking or eating.

In the case of using the bacterium belonging to *Bifidobacterium pseudocatenulatum* according to the present invention, the dose is not strictly limited and is preferably from 10⁵ cfu to 10¹³ cfu, particularly preferably from 108 cfu to 10¹² cfu, per day in terms of the number of viable bacteria.

### Examples

Next, the present invention will be described in more detail with reference to Examples. However, the present invention is not limited to these examples by any means.

### Example 1 Preparation of bacterium belonging to Bifidobacterium pseudocatenulatum having xylanase gene and having excellent viability in milk component-containing medium - 1

*Bifidobacterium pseudocatenulatum* YIT 11057 was bred as described below as a parent strain, and the number of viable bacteria (proliferative activity) and viability in a milk component-containing medium were confirmed.

### (1) Breeding

1% of a frozen bacterial liquid of YIT 11057 was inoculated to modified GAM (mGAM) liquid medium (Nissui Pharmaceutical Co., Ltd.) and statically cultured at 37°C for 4 hours under anaerobic conditions, and the bacterial liquid was diluted 10-fold with PBS. The diluted bacterial liquid was added dropwise to the center of a Petri dish and irradiated with ultraviolet ray under conditions (wavelength: 254 nm, illuminance: approximately 0.2 mW/cm², 60 sec) which attained a survival rate on the order of approximately 1% after ultraviolet irradiation in preliminary study. The UV-irradiated bacterial liquid was subcultured in a mother medium (12% skim milk powder, 0.1% yeast extracts, 0.03% L-cysteine hydrochloride monohydrate, 0.2% calcium carbonate, sterilization conditions: 115°C for 20 min), statically cultured at 37°C for 24 hours, and then refrigerated at 4°C or 10°C for 2 weeks, and this operation was repeated 18 times (36 weeks). The refrigeration was performed in the mother medium under anaerobic conditions with Anaeropack (Mitsubishi Gas Chemical Co., Inc.).

The bacterial liquid thus refrigerated was spread at an appropriate concentration over mGAM agar plate medium supplemented with 1% lactose, which was adjusted to pH 4.4 or 4.6 with 4 M hydrochloric acid, and statically cultured at 37°C for 14 days under anaerobic conditions. Then, a colony was picked up and inoculated to mGAM liquid medium supplemented with 1% lactose. The colony was statically cultured at 37°C for 20 hours under anaerobic conditions, spread over TOS propionate agar plate medium, and statically cultured at 37°C for approximately 48 hours under anaerobic conditions, followed by colony pickup and inoculation to mGAM liquid medium supplemented with 1% lactose. This operation was repeated twice to obtain *Bifidobacterium pseudocatenulatum* Y 51493.

### (2) Confirmation of the number of viable bacteria and survival rate

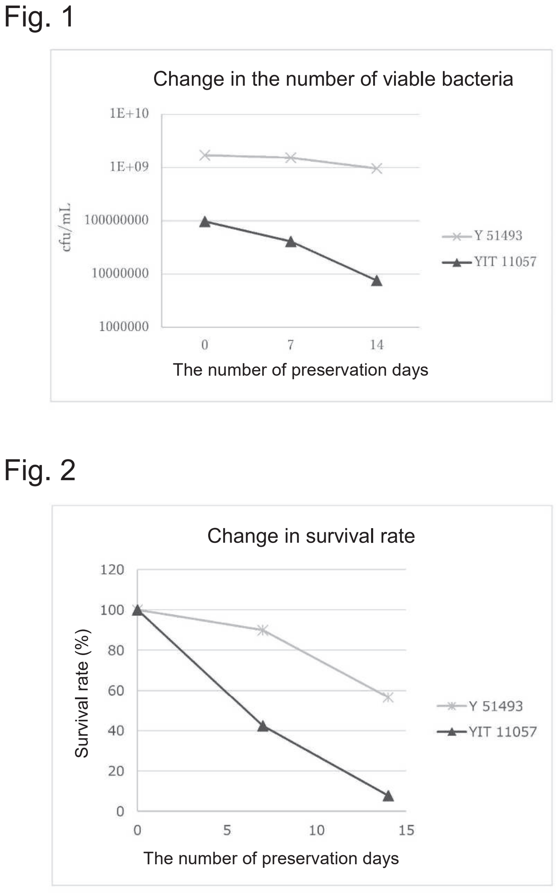

In the study of viability, a bacterial liquid was used which was obtained by inoculating a frozen bacterial liquid of each of the bacterial strains YIT 11057 and Y 51493 to the mother medium, performing preculture at 37°C for 24 hours, then inoculating 5% of the bacterial liquid to mother medium, and performing main culture at 37°C for 24 hours, followed by refrigeration at 4°C (0, 7, and 14 days). The bacterial liquid of each of YIT 11057 and Y 51493 serially diluted with saline was spread over TOS propionate agar plate medium and cultured at 37°C for 48 hours under anaerobic conditions, and the number of viable bacteria was measured. A survival rate was calculated from the measured number of viable bacteria. The survival rate is indicated in percentage of the measured number of viable bacteria divided by the number of viable bacteria in 0-day preservation. The results are shown in Figures 1 and 2. As a result, it could be confirmed that Y 51493 obtained by breeding was excellent in both the number of viable bacteria (proliferative activity) and viability in a milk component-containing medium, as compared with YIT 11057.

### Reference Example 1 Finding of genomic mosaicism in bacterium belonging to genus Bifidobacterium

The analysis of the ability to utilize indigestible polysaccharide and genomic analysis using bacterial strains belonging to *Bifidobacterium pseudocatenulatum* have revealed that some bacterial strains exhibit the ability to utilize long-chain xylans, which is defined by xylanase (BpXyn10A) gene residing in the chromosome; and xylanase gene-possessing strains, when plotted to a phylogenetic tree prepared on the basis of genomic information of bacterial strains belonging to *Bifidobacterium pseudocatenulatum,* are dispersed throughout the phylogenetic tree, rather than being concentrated at a particular cluster (Non Patent Literature 1). This suggested that the xylanase gene is horizontally transferred between bacterial strains. Accordingly, the xylanase gene was tested for its horizontal transfer *in vitro.*

In general, horizontal gene transfer in bacterium is known to have three patterns: natural transformation by the uptake of extracellular DNA, transduction by phage infection, and conjugation mediated by the transmission of plasmids or the like. Accordingly, in order to enable detection of all the events, two strains of *Bifidobacterium pseudocatenulatum* differing in phenotype were individually cultured and then cocultured for a given time on an agar medium to test horizontal gene transfer.

### (1) Bacterial strain used

Tetracycline (Tc)-sensitive *Bifidobacterium pseudocatenulatum* strains having the xylanase gene (YIT 11025, YIT 11027, YIT 12242, YIT 12820, YIT 12824, and YIT 12986) were used as donors, and Tc-resistant *Bifidobacterium pseudocatenulatum* strains having no xylanase gene (YIT 4072^{T}, YIT 11955, YIT 11956, YIT 12228, YIT 12818, YIT 12822, and YIT 12987) were used as recipients.

### (2) Methods for testing horizontal transfer of xylanase gene

Each of the donor and recipient bacterial strains was inoculated to modified GAM (mGAM) liquid medium (Nissui Pharmaceutical Co., Ltd.) supplemented with 0.5% by mass of glucose/lactose, and cultured until the logarithmic growth phase. 200 µL each of the donor and recipient bacterial liquids was mixed (the number of bacteria: approximately 10⁸⁻⁹ organisms) and centrifuged, followed by the removal of a supernatant using a 200 µL pipette. Pellets were suspended using a very small amount of the medium remaining in a tube, and the whole amount of the suspension was then left standing on mGAM agar medium (Nissui Pharmaceutical Co., Ltd.). After being anaerobically cultured at 37°C for 16 to 24 hours, bacterial cells were scraped off using a 1 µL loop and inoculated to mPY-AX-Tc (mPY medium; see Non Patent Literature 1, arabinoxylan (AX): final concentration: 0.5% by mass, Tc: final concentration: 10 µg/mL) liquid medium in which the donor alone or the recipient alone was unable to proliferate. Turbidity was monitored using Eon Microplate Spectrophotometers (BIOTEC Co., Ltd.). Some combinations found to elevate turbidity in the selective medium were subcultured in a fresh medium having the same composition as above to confirm the reproducibility of elevation in turbidity. This bacterial liquid was smeared over Tc-containing (final concentration: 10 µg/mL) mGAM agar medium and then anaerobically cultured overnight at 37°C, and the resulting single colony was subcultured in the same agar medium as above. On the next day, some bacterial cells were inoculated to mPY-AX-Tc liquid medium, while some bacterial cells were suspended in TE buffer, heated at 95°C for 5 minutes, and used as a simple template for PCR. The presence or absence of proliferation in mPY-AX-Tc medium was confirmed, and when the recipient was YIT 11956, bacterial strain identification and the confirmation of the presence or absence of xylanase gene were performed by PCR using recipient (YIT 11956)-specific primers (BP28-f: GGGCAAGATCGGCATCATCTA (SEQ ID NO: 10), BP28-r: CACATTGGTGGTGTTCACGTC (SEQ ID NO: 11)) and xylanase gene-specific primers (pBpXyn10A-F: CGAGAATGCGAACACGTACTTC (SEQ ID NO: 12), pBpXyn10A-R: CTGCTCGGTGTTGTAATCGTTG (SEQ ID NO: 13)). A colony confirmed to contain the xylanase gene was cryopreserved.

### (3) Genomic analysis method

DNA was extracted from the bacterial cells obtained in (2), and a short read sequence and a long read sequence were obtained using a second-generation sequencer Miseq (Illumina, Inc.) and a third-generation sequencer MinION (Oxford Nanopore Technologies plc.). A full-length genomic sequence was obtained by hybrid assembly. The genomic sequences of the donor strain, the recipient strain, and the strain that acquired the gene were aligned by snippy (https://github.com/tseemann/snippy), and the alignment results were input to fastGEAR (Mostowy et al., Molecular Biology and Evolution 34 (5), 1167-1182) to identify a homologous recombination region. Information on single-nucleotide polymorphisms (SNPs) was extracted from the genomic sequences using nucmer, delta-filter and show-snps of MUMMER (Kurtz et al., Genome Biology 5, R12, 2004) package. The format of an output file was adjusted by custom script, and the homologous recombination region was then visualized using Artemis ACT (Sanger Institute).

### (4) Testing results about xylanase horizontal gene transfer

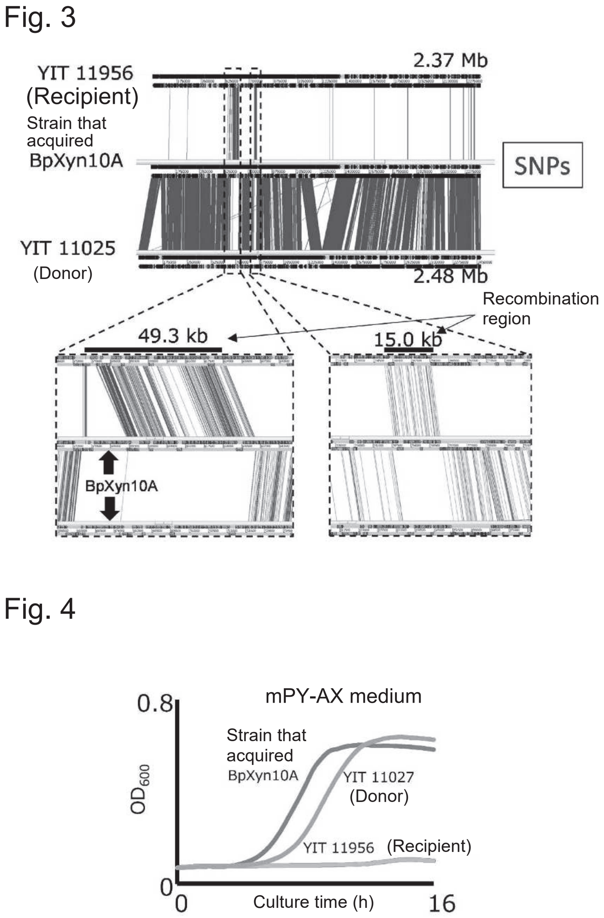

A donor and a recipient were mixed, then anaerobically cultured overnight in mGAM agar medium, and inoculated to mPY-AX-Tc liquid selective medium, and proliferative activity was monitored. As a result, a plurality of donor-recipient combinations were found to elevate turbidity. As a result of genomic analysis on 13 strains that acquired the gene and were obtained with YIT 11956 or YIT 4072^{T} as a recipient and YIT 11025, YIT 11027, YIT 12820, or YIT 12824 as a donor from among the combinations, all the strains that acquired the gene were confirmed to have the xylanase gene and to be very closely related to the recipient, not the donor. The xylanase gene on the chromosome was found to be transmitted from the donor which was a xylanase gene-possessing strain to the recipient which was a xylanase gene non-possessing strain (Figure 3 and Table 1). The strain having the transmitted xylanase gene was confirmed to be able to proliferate in mPY-AX medium (Figure 4).

As a result of comparing genomic sequences in detail at the SNPs level, SNPs in regions from 16.5 to 247 kb including the newly acquired xylanase gene as well as upstream and downstream sites thereof in the strain that acquired the gene were consistent with the donor. Specifically, double crossover homologous recombination was found to occur extensively in the strain that acquired the gene, thereby causing replacement with donor-derived genomic regions including the xylanase gene and upstream and downstream sites thereof and the horizontal transfer of the xylanase gene. The replacement of the genomic regions occurred in a mosaic pattern throughout the genome, and replacement regions also differed among bacterial strains. Specifically, donor-derived genomic regions were integrated at seven locations at maximum per strain and in regions from 0.5 to 247 kb (Figure 3). On the other hand, any trace of phages or plasmids was not observed in the replacement regions.

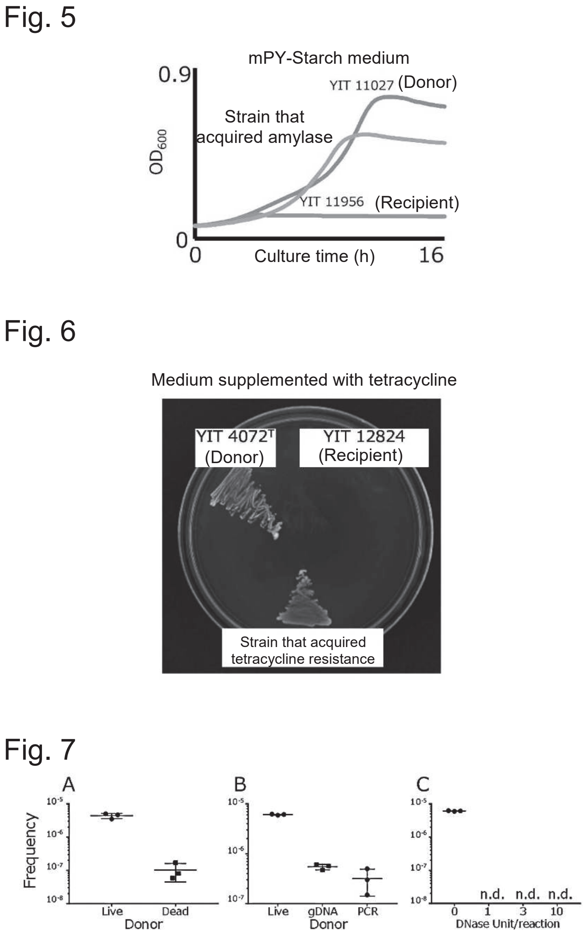

In order to test the generality of this phenomenon, tests were also attempted to horizontally transfer other genes possessed by *Bifidobacterium pseudocatenulatum.* First, attention was made on amylase gene which defines an ability to utilize starch. A Tc-sensitive *Bifidobacterium pseudocatenulatum* strain having the amylase gene (YIT 11027) was used as a donor, and a Tc-resistant *Bifidobacterium pseudocatenulatum* strain having no amylase gene (YIT 11956) was used as a recipient. When bacterial cells after mixing of the strains were inoculated to mPY-St-Tc (starch (St): final concentration: 0.5% by mass, Tc: final concentration: 10 µg/mL) liquid medium by the same approach as in (2), a strain having the horizontally transferred amylase gene was confirmed to appear by genomic mosaicism (Table 1). The strain having the transmitted amylase gene was confirmed to be able to proliferate in mPY-Starch medium (Figure 5). Further attention was made on Tc resistance gene. Erythromycin (Em)-sensitive *Bifidobacterium pseudocatenulatum* strains having the Tc resistance gene (YIT 4072^{T}, YIT 11955, and YIT 12987) were used as donors, and an Em-resistant *Bifidobacterium pseudocatenulatum* strain having no Tc resistance gene (YIT 12824) was used as a recipient. When bacterial cells after mixing of the strains were smeared over agar medium of modified GAM supplemented with 0.5% by mass of lactose (mGAML) and supplemented with Em (final concentration: 1 µg/mL)-Tc (final concentration: 10 µg/mL) (EmTc-mGAML) by the same approach as in (2), a strain having the horizontally transferred Tc resistance gene was confirmed to appear by genomic mosaicism (Table 1). The strain having the transmitted Tc resistance gene was confirmed to be able to proliferate in Tc-mGAML medium (Figure 6).

**[Table 1]**

| Donor strain | | Recipient strain | | Acquired phenotype |
|---|---|---|---|---|
| LCX⁺ | Tc^{S} | LCX⁻ TC^{R} | | |
| | YIT 11025 | | YIT 11956 | LCX⁺ Tc^{R} |
| | YIT 11027 | | YIT 11956 | LCX⁺ Tc^{R} |
| | YIT 12820 | | YIT 11956 | LCX⁺ Tc^{R} |
| | YIT 12824 | | YIT 11956 | LCX⁺ Tc^{R} |
| | YIT 12824 | | YIT 4072⁷ | LCX⁺ Tc^{R} |

| ST⁺ Tc^{S} | | ST⁻ TC^{R} | | |
|---|---|---|---|---|
| | YIT 11027 | | YIT 11956 | ST⁺ Tc^{R} |

| Em^{S}Tc^{R} | | Em^{R} Tc^{S} | | |
|---|---|---|---|---|
| | YIT 11955 | | YIT 12824 | Em^{R} Tc^{R} |
| | YIT 12987 | | YIT 12824 | Em^{R} Tc^{R} |
| | YIT 4072^{T} | | YIT 12824 | Em^{R} Tc^{R} |

| | | | | |
|---|---|---|---|---|
| * LCX^{+/-}: Having BpXyn10A gene, presence/absence of ability to utilize long-chain xylans (LCX) * Tc^{S/R}: Tetracycline sensitivity/resistance * ST^{+/-}: Presence/absence of ability to utilize starch * Em^{S/R}: Erythromycin sensitivity/resistance | | | | |

### Reference Example 2 Test of natural transformation in bacterium belonging to genus Bifidobacterium

The genomic mosaicism of the bacterium belonging to the genus *Bifidobacterium* was tested for whether it is ascribable to natural transformation because any trace of horizontal transfer of phages or plasmids was not observed therein.

### (1) Bacterial strain used

A Tc-resistant and Em-sensitive *Bifidobacterium pseudocatenulatum* type strain YIT 4072^{T} was used as a donor, and Tc-sensitive and Em-resistant *Bifidobacterium pseudocatenulatum* YIT 12824 was used as a recipient.

### (2) Method for extracting genomic DNA for use as donor and preparing PCR product

YIT 4072^{T} was cultured overnight in mGAML liquid medium, and high-molecular-weight genomic DNA was purified using NucleoBond Buffer Set III and NucleoBond AXG20 column (Takara Bio Inc.). The double-stranded DNA was quantified using Picogreen kit, and diluted to 30 ng/µL with TE buffer. By using this genomic DNA as a template, a region of approximately 20 kb comprising the Tc resistance gene (tetW) was amplified by PCR. A high-fidelity enzyme PrimeSTAR GXL DNA polymerase (Takara Bio Inc.) was used as a PCR enzyme. 0.2 µL of the enzyme, 0.04 µL each of 50 µM primers (SEQ ID NO: 14 and 15), 1 µL of the template DNA (0.02 ng/µL), 1.6 µL of a dNTP solution, and 4 µL of 5 × buffer were mixed, and the amount of a reaction solution per PCR tube was set to 20 µL. The obtained PCR products were precipitated in ethanol and then dissolved in TE buffer. Double-stranded DNA was quantified using Picogreen and then diluted to 1 ng/µL with TE buffer, and the dilution was divided in small portions and cryopreserved.

Table 2 shows the primers and PCR conditions used in this Example.

**[Table 2]**

| Primer name | Sequence (5'-3') | SEQ ID NO | Amplified product length (bp) | PCR conditi ons |
|---|---|---|---|---|
| BpTy20k-F | TCCTGTGGATTTCGACGGTGACGAGCAG | 14 | 19645 | 1 |
| BpTy20k-R | GAGTGCGGTT GATGGTGCATACGGCAAG | 15 | | |

| | | | | |
|---|---|---|---|---|
| PCR conditions 1.98°C 20sec, (98°C 10sec, 68°C 10 min)×30, 4°C ∞ | | | | |

### (3) Method for preparing dead bacterial cell

YIT 4072^{T} was cultured in 2 mL of mGAML liquid medium until the late logarithmic growth phase, and washed with mPY liquid medium, and pellets were then suspended in 180 µL of the same medium as above. A 20 µL aliquot of the suspension was collected into a 1.5 mL screw cap tube and heated at 80°C for 10 minutes using a heat block to prepare dead bacterial cells. This treatment was separately confirmed to completely kill the bacterial cells.

### (4) Test of horizontal transfer of antibiotic resistance gene with viable bacterial cell, heat-killed bacterial cell, and purified DNA as donors

Frozen bacterial cells of each of the bacterial strains YIT 4072^{T} and YIT 12824 were inoculated to mGAML agar medium and anaerobically cultured at 37°C, and the obtained bacterial cells were then subcultured in 400 µL of the same liquid medium as above. After culture for 3 to 4 hours, 1600 µL of the same liquid medium as above was further added thereto, and the bacterial cells were cultured for 4 to 5 hours until the culture was from the late logarithmic growth phase to the stationary phase. Each bacterial liquid was harvested, washed with mPY liquid medium, then harvested again, and suspended in 180 µL of the same medium as above. 5 µL of the bacterial liquid of the recipient (YIT 12824) and 5 µL of the viable bacterial liquid of the donor (YIT 4072^{T}), the dead bacterial liquid prepared in (3), or the purified DNA prepared in (2) were mixed (the number of bacteria: approximately 10⁷⁻⁸ organisms each), and the whole amount of the mixture was left standing on mGAM agar medium. After being anaerobically cultured at 37°C for 16 to 24 hours, the whole amount of the bacterial cells was scraped off using a 1 µL loop, suspended in 60 µL of mPY liquid medium, serially diluted, smeared over EmTc-mGAML agar medium, and anaerobically cultured at 37°C for 2 to 3 days, followed by the counting of the number of colonies. Some colonies were subjected to genomic analysis in the same manner as in Example 1(3) to confirm that homologous recombination occurred.

### (5) Test of influence of addition of DNA-degrading enzyme (DNase I)

Washed viable bacterial liquids of YIT 4072^{T} and YIT 12824 were prepared in the same manner as in (4). 20 µL aliquots of the respective washed bacterial liquids of the strains were collected into a 1.5 mL tube, mixed, and centrifuged, and pellets were then suspended in 40 µL of 1 × DNase I buffer (Takara Bio Inc.). 8 µL of an undiluted liquid (5 U/µL) of Recombinant DNase I (Takara Bio Inc.) or 1/10 dilution thereof (0.5 U/µL) or 3/10 dilution thereof (1.5 U/µL) with 1 × DNase I buffer was added to the suspension. 12 µL each of three samples per condition of the mixture was left standing on mGAM agar medium. The agar medium on which the bacterial cells were left standing was anaerobically cultured at 37°C for 16 hours. Then, the whole amount of the bacterial cells was scraped off using a 1 µL loop, suspended in 60 µL of mPY liquid medium, and then appropriately diluted using the same medium as above. In order to measure the number of bacteria having the horizontally transferred Tc resistance gene, the bacterial liquid was smeared over EmTc-mGAML agar medium. In order to detect the total number of viable bacteria of the Em-resistant strain, the bacterial liquid was also smeared over Em-mGAML agar medium. Each agar medium was anaerobically cultured at 37°C for 2 to 3 days. The ratio of the number of bacteria having mosaicism to the total number of viable bacteria of the Em-resistant strain was regarded as the frequency of mosaicism.

### (6) Results

Viable bacterial cells of donor YIT 4072^{T} (Tc-resistant strain) and recipient YIT 12824 (Em-resistant strain) were mixed, anaerobically cultured overnight in mGAM agar medium, and studied for whether a strain resistant to both the antibiotics appeared in the selective medium in which the donor alone or the recipient alone was unable to proliferate. As a result, colonies appeared which exhibited resistance to both the antibiotics. As a result of genomic analysis on six strains thus obtained, homologous recombination was confirmed to occur in a mosaic pattern in a plurality of regions including the Tc resistance gene and its neighboring regions. The number of recombination regions was from 1 to 4, and the maximum recombination region length was from 22 to 112 kb.

In the case of using dead bacterial cells as a donor, the horizontal transfer of the Tc resistance gene was confirmed, though the frequency was decreased as compared with the case of using the viable bacterial cells as a donor (Figure 7A). Likewise, horizontal transfer occurred even if the donor was exchanged to genomic DNA or PCR products (Figure 7B). In the case of using the dead bacterial cells as a donor, the number of recombination regions was from 1 to 2, and the maximum recombination region length was from 16 to 45 kb, in two strains subjected to genomic analysis. In the case of using the genomic DNA as a donor, the number of recombination regions was 1, and the maximum recombination region length was from 12 to 16 kb, in two strains subjected to genomic analysis. In the case of using, as a donor, the PCR products prepared with the genomic DNA of YIT 4072^{T} as a template, the number of recombination regions was 1, and the maximum recombination region length was 12 kb, in one strain subjected to genomic analysis. As a result of further adding a DNA-degrading enzyme to the test system using the viable bacterial cells as a donor, horizontal gene transfer was inhibited (Figure 7C). This revealed that the pathway of genomic mosaicism involving horizontal gene transfer in the bacterium belonging to the genus *Bifidobacterium* was natural transformation by the uptake of extracellular DNA and homologous recombination. Accordingly, the frequency of mosaicism described above means a transformation frequency.

As a result of confirming the universality of natural transformation in *Bifidobacterium pseudocatenulatum,* the horizontal transfer of the Tc resistance gene was newly confirmed in 11 strains by using, as a donor, PCR products prepared with the genomic DNA of YIT 4072^{T} as a template (Table 3).

**[Table 3]**

| Donor strain | | Recipient strain | Acquired phenotype |
|---|---|---|---|
| Tc^{R} | | Tc^{S} | |
| | YIT 4072^{T} (PCR product) | YIT 12989 | Tc^{R} |
| | YIT 4072^{T} (PCR product) | YIT 11952 | Tc^{R} |
| | YIT 4072^{T} (PCR product) | YIT 11953 | Tc^{R} |
| | YIT 4072^{T} (PCR product) | YIT 12145 | Tc^{R} |
| | YIT 4072^{T} (PCR product) | YIT 12203 | Tc^{R} |
| | YIT 4072^{T} (PCR product) | YIT 12232 | Tc^{R} |
| | YIT 4072^{T} (PCR product) | YIT 12817 | Tc^{R} |
| | YIT 4072^{T} (PCR product) | YIT 12819 | Tc^{R} |
| | YIT 4072^{T} (PCR product) | YIT 12820 | Tc^{R} |
| | YIT 4072^{T} (PCR product) | YIT 12821 | Tc^{R} |
| | YIT 4072^{T} (PCR product) | YIT 12985 | Tc^{R} |

| | | | |
|---|---|---|---|
| * Tc^{S/R}: Tetracycline sensitivity/resistance | | | |

### Example 2 Preparation of bacterium belonging to Bifidobacterium pseudocatenulatum having xylanase gene and having excellent viability in milk component-containing medium - 2

Y 51493 obtained in Example 1 lost the ability to utilize glucose during the course of breeding (see Table 4 below). As a result of genomic analysis, Y 51493 underwent single-nucleotide deletion of a C base at position 369 of a gene (SEQ ID NO: 9) encoding a glucose transporter (beta-glucoside PTS system EIICBA component). Therefore, frameshift was considered to largely change the amino acid sequence and consequently cause glucose to be no longer transported into cells. It is desirable to maintain the ability to utilize glucose from the viewpoint of carbohydrate metabolism in the intestines. On the other hand, the restoration of base deletion by conventional UV irradiation or long-term subculture is not realistic. Accordingly, in order to restore the single-nucleotide deletion of the glucose transporter gene, Y 51493 as a parent strain, i.e., a recipient, and the genomic DNA of YIT 11057 as donor DNA were subjected to the transformation method of Reference Example 2.

### (1) Bacterial strain used

Y 51493 prepared in Example 1 was used as a recipient.

### (2) Donor DNA

YIT 11057 was cultured overnight in mGAML liquid medium, and genomic DNA was extracted from 1 mL of the resultant bacterial liquid by the bead phenol method and used.

### (3) Transformation and selection of bacterial cell having restored ability to utilize glucose

All the operations were performed in an anaerobic glove box. Frozen bacterial cells of Y 51493 were inoculated to mGAML agar medium and anaerobically cultured overnight. On the next morning, a plurality of colonies were scraped off at the same time and subcultured in 400 µL of the same liquid medium as above. After culture for 3 to 4 hours, 1600 µL of the same liquid medium as above was further added thereto, and the bacterial cells were cultured for 4 to 5 hours. After centrifugation, bacterial cell pellets were washed once with 500 µL of mPY liquid medium, and the pellets were suspended in 180 µL of the same liquid medium as above. The bacterial liquid and a DNA solution (genomic DNA of YIT 11057 precipitated in ethanol and then dissolved in TE buffer) were mixed in equal volumes, and 10 µL of the mixed solution was spotted onto mGAM agar medium supplemented with 50 mM magnesium chloride, and left standing for several minutes until the liquid was dried, followed by anaerobic culture at 37°C. The agar medium used was sterilized by autoclaving on the day of use. 16 hours later, the whole amount of bacterial cell pellets was scraped off using a 1 µL loop and suspended in 500 µL of mPY liquid medium. A 50 µL aliquot of this suspension was collected and suspended in 640 µL of mPY liquid medium supplemented with 0.5% glucose. A 200 µL aliquot of this suspension was collected into each well of a 96-well plate, 50 µL of mineral oil was layered thereon, and then a proliferation curve was obtained using a microplate reader PowerWave 340 (BIOTEC Co., Ltd.). The remaining bacterial liquid was anaerobically cultured at 37°C in a 1.5 mL tube. When elevation in turbidity was confirmed on a PC screen, 10 µL of the bacterial liquid in the 1.5 mL tube was subcultured in 500 µL of a fresh liquid medium having the same composition as above and divided into a 96-well plate and a 1.5 mL tube, and proliferation was monitored again. When proliferation was confirmed, the bacterial liquid in the 1.5 mL tube was then smeared by streak over mGAML agar medium. A single colony was isolated, then suspended in glycerol, and preserved at -80°C. The bacterial cells of the isolated strain were suspended in mPY liquid medium supplemented with 0.5% glucose to confirm proliferative activity. The full-length genome of the isolated strain was determined by the procedures described in Reference Example 1(3).

### (4) Test of ability to utilize carbohydrate

Frozen bacterial cells of the isolated strain obtained in (3), Y 51493, and YIT 11057 were each inoculated to modified GAM agar medium supplemented with 1% lactose, and then cultured for 2 to 3 days in an anaerobic glove box. A plurality of obtained colonies were scraped off and subcultured in the same liquid medium as above. After culture overnight at 37°C, 5% of the culture was subcultured in a fresh medium having the same composition as above, and cultured for 6 to 9 hours until reaching the middle and late logarithmic growth phase. 2 µL of the culture supplemented with PIPES-ILS medium so as to attain OD₆₀₀ = 0.2 was added to 198 µL of PIPES-ILS medium supplemented with 0.5% of each carbohydrate dispensed to each well of a 96-well plate, and 50 µL of mineral oil was then layered thereon, followed by culture at 37°C under anaerobic conditions. When the amount of increase in OD₆₀₀ was 0.1 or more 72 hours later, it was determined to be the presence of the ability to utilize the carbohydrate.

### (5) Confirmation of the number of viable bacteria and viability

The number of viable bacteria and a survival rate were calculated for the isolated strain obtained in (3) and Y 51493 by the same method as in Example 1(2) except that refrigeration was performed for 0, 14, 21, and 28 days.

### (6) Analysis on mutation site

A mutation which occurred in the bacterial strain obtained in (3) was searched for with the full-length genome of YIT 11057 as a reference. First, single-nucleotide substitution (SNPs) was identified using snippy. Large-scale genomic change and SNPs which were not detected by snippy were visually confirmed using mauve (Darling et al., Genome Research 14, 1394-1403).

### (7) Results

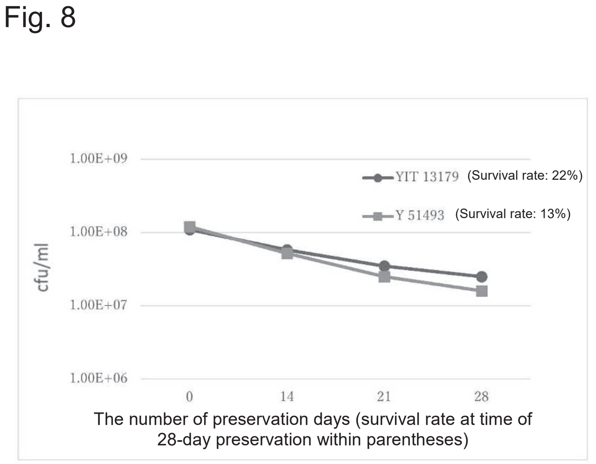

As a result of performing transformation using DNA extracted from YIT 11057 as a donor and viable bacterial cells of Y 51493 as a recipient, a transformant *Bifidobacterium pseudocatenulatum* YIT 13179 was obtained which proliferated in a liquid medium having glucose as only one carbohydrate source. As a result of confirming the ability of YIT 13179 to utilize glucose, this strain was confirmed to utilize glucose (Table 4). Both YIT 13179 and Y 51493 had a survival rate of 40% or more when preserved for 2 weeks, and YIT 13179 was confirmed to have an improved survival rate as compared with Y 51493 (Figure 8).

The full-length genome of YIT 13179 was determined, and the nucleotide sequence of glucose transporter gene was confirmed. As a result, single-nucleotide deletion which occurred in Y 51493 was restored in YIT 13179, which thus had the full-length sequence of the gene identical to that of YIT 11057.

**[Table 4]**

| day3 (72h) | | Soluble starch | Wheat arabinoxylan | Lactose | Glucose |
|---|---|---|---|---|---|
| *B.pseudocatenulatum* | YIT 11057 | 0.78 | 0.38 | 0.93 | 1.04 |
| | Y 51493 | 0.71 | 0.37 | 0.88 | 0.01 |
| | YIT 13179 | 0.86 | 0.58 | 1.05 | 0.92 |

Y 51493, YIT 11057, and YIT 13179 had almost equivalent ability to utilize arabinoxylan, starch, and lactose (Table 4).

Mutations were introduced to 15 locations on the genome during the course of breeding from YIT 11057 to Y 51493. Thus, these mutations are presumed to contribute to improvement in viability in a milk medium. In YIT 13179, only one of these locations was restored to the sequence of YIT 11057. The restored mutation was the deletion of the glucose transporter gene.

Four mutations, which were absent in Y 51493 compared with YIT 11057, newly occurred in YIT 13179 but included no gene presumed to be related to safety. Although a harmful gene was further searched for using MiFuP Safety, any gene which raised concern about safety was not detected in YIT 13179.

## Claims

1. A bacterium belonging to *Bifidobacterium pseudocatenulatum,* the bacterium having xylanase gene on a genome and having higher viability in a milk component-containing medium than *Bifidobacterium pseudocatenulatum* YIT 11057 (NITE BP-02930).

2. The bacterium according to claim 1, wherein the xylanase gene is a polynucleotide consisting of the nucleotide sequence represented by SEQ ID NO: 1 or a polynucleotide having 70% or more identity to the nucleotide sequence and encoding a protein having xylanase activity.

3. The bacterium according to claim 1, wherein a survival rate is 20% or more when the bacterium is cultured in the milk component-containing medium for 24 hours and then preserved at a low temperature for 2 weeks.

4. *Bifidobacterium pseudocatenulatum* Y 51493 (NITE ABP-03852), *Bifidobacterium pseudocatenulatum* YIT 13179 (NITE ABP-03853), or a bacterial strain closely related thereto.

5. A food or drink product comprising the bacterium according to any one of claims 1 to 4.

6. The food or drink product according to claim 5, which is a fermented milk food or drink product.
